(19) Europäisches Patentamt
European Patent Office
Office européen des brevets

(11) Veröffentlichungsnummer: **0 250 903 B1**

(12) # EUROPÄISCHE PATENTSCHRIFT

(45) Veröffentlichungstag der Patentschrift: **27.11.91**

(21) Anmeldenummer: **87108007.3**

(22) Anmeldetag: **12.09.83**

(51) Int. Cl.[5]: **C07D 471/08**, C07D 471/10, //(C07D471/08,221:00,221:00), (C07D471/10,221:00,221:00)

(60) Veröffentlichungsnummer der früheren Anmeldung nach Art. 76 EPÜ: **0 103 833**

(54) **Tetraoxodiazabicyclo-(3,3,1)-nonane.**

(30) Priorität: **18.09.82 DE 3234697**

(43) Veröffentlichungstag der Anmeldung: **07.01.88 Patentblatt 88/01**

(45) Bekanntmachung des Hinweises auf die Patenterteilung: **27.11.91 Patentblatt 91/48**

(84) Benannte Vertragsstaaten: **AT BE CH DE FR GB IT LI LU NL SE**

(56) Entgegenhaltungen: **FR-A- 2 375 235**

(73) Patentinhaber: **Kali-Chemie Pharma GmbH**
**Hans-Böckler-Allee 20 Postfach 220**
**W-3000 Hannover 1(DE)**

(72) Erfinder: **Schön, Uwe, Dr. Dipl.-Chem.**
**Föhrenkamp 12**
**W-3167 Burgdorf(DE)**
Erfinder: **Hachmeister, Bernd, Dipl.-Chem., Dr. rer. nat.**
**Mövenkamp 4**
**W-3004 Isernhagen 1(DE)**
Erfinder: **Kehrbach, Wolfgang, Dipl.-Chem., Dr. rer. nat.**
**Altenbekener Damm 41**
**W-3000 Hannover 1(DE)**
Erfinder: **Kühl, Ulrich, Dr. med. vet.**
**Franzburger Strasse 10**
**W-3007 Gehrden 1(DE)**
Erfinder: **Buschmann, Gerd, Dr. med. vet.**
**Ernst-Ebeling-Strasse 9**
**W-3000 Hannover 72(DE)**

(74) Vertreter: **Lauer, Dieter, Dr.**
**c/o Kali-Chemie AG Postfach 220 Hans-Böckler-Allee 20**
**W-3000 Hannover 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue Tetraoxodiazabicyclo-(3,3,1)-nonane, Verfahren zu ihrer Herstellung und ihre Verwendung als Zwischenprodukte zur Herstellung von pharmakologisch aktiven N,N'-disubstituierten Diazabicyclo-(3,3,1)-nonanen.

Mehrere Veröffentlichungen über 3,7-Diazabicyclo(3,3,1)-nonane sprechen für ein großes Interesse in chemischer (z.B. Russian. Chem. Rev. 34, 439 (1965), Anm. Ist. Super Sanita 4, 157 (1968), J. Org. Chem. 33, 355 (1968), Russian Chem. Rev. 42, 190 (1973), Chem. Ber. 110, 3894 ff, Austral. J. Chem. 13 (1960), 129 ff, J. Chem. Soc. 115 (1919), S. 686 ff) und pharmakologischer Hinsicht (z.B. Eur. J. med. Chem. 1977, S. 301-5). Die pharmakolgischen Wirkungen reichen von einem geringen lokalanaesthetischen Effekt in J. Chem. Soc. 1951, 1706 über eine Wirkung auf das zentrale Nervensystem in DE-OS 26 58 558 und DE-OS 27 49 584 bis hin zu antiarrhythmischen Eigenschaften in DE-OS 24 28 792, DE-OS 27 26 571 und DE-OS 27 44 248.

Aufgabe der Erfindung ist es, neue Tetraoxodiazabicyclo-(3,3,1)-nonane zur Verfügung zu stellen, die wertvolle Zwischenprodukte zur Herstellung von pharmakolgisch wirksamen neuen Diazabicyclo-(3,3,1)-nonanen darstellen. Die Aufgabe wird gelöst durch die vorliegende Erfindung.

Die Erfindung betrifft neue Verbindungen der allgemeinen Formel (I)

(s. Formel I)

in der
$R_1$ und $R_2$ unabhängig voneinander Alkyl mit 1 bis 12 C-Atomen, Alkenyl mit 2 bis 12 C-Atomen oder Cycloalkyl mit 3 bis 6 C-Atomen, wobei das Cycloalkyl entweder direkt oder über einen Alkylenrest von 1 bis 3 C-Atomen, vorzugsweise 1 C-Atom mit dem N-Atom verbunden ist, bedeuten und
$R_3$ und $R_4$ entweder unabhängig voneinander Alkyl mit 1 bis 7 C-Atomen bedeuten oder gemeinsam eine Alkylenkette $-(CH_2)_n-$ bilden, mit n = 3 bis 6, mit der Maßgabe, daß die Reste $R_1$ bis $R_4$ zusammengezählt mindestens 5 Kohlenstoffatome enthalten.

Erfindungsgemäß werden die Verbindungen der allgemeinen Formel (I) erhalten, indem man substituierte Dinitrile der allgemeinen Formel (III)

(s. Formel III)

oder Mononitrile (IV)

(s. Formel IV)

in denen $R_3$ und $R_4$ obige Bedeutung besitzen und $R_5$ die für $R_1$ angegebene Bedeutung besitzt oder Wasserstoff bedeutet, sauer hydrolysiert zu den Verbindungen (V)

(s. Formel V)

worin $R_5$ , $R_3$ und $R_4$ obige Bedeutung besitzen, und diese anschließend für den Fall, daß $R^5$ die Bedeutung von $R^1$ hat, mit Verbindungen der allgemeinen Formel (II)

(s. Formel II)

worin $R_6$ die für $R_1$ und $R_2$ angegebene Bedeutung hat und X eine Fluchtgruppe bedeutet, umsetzt zu Verbindungen der allgemeinen Formel (Ib)

(s. Formel Ib)

worin $R_1$ , $R_3$ , $R_4$ und $R_6$ obige Bedeutung besitzen,
oder
für den Fall, daß $R_5$ in den Verbindungen (V) Wasserstoff ist, diese Verbindungen mit Verbindungen der Formel (II) umsetzt zu symmetrisch substituierten Verbindungen (Ic)

(s. Formel Ic)

worin $R_3$ , $R_4$ und $R_6$ obige Bedeutung besitzen.

In einer bevorzugten Gruppe der Verbindungen der Formel (I) enthalten die Substituenten $R_1$ und/oder $R_2$ jeweils bis zu 12, vorzugsweise bis 7 Kohlenstoffatome.

Soweit unter $R_1$ und $R_2$ Alkyl verstanden wird, kommt sowohl verzweigtes als auch unverzweigtes Alkyl in Frage. Unverzweigte Alkylreste sind der Methyl-, Athyl-, n-Propyl-, n-Butyl-, n-Pentyl-, n-Hexyl- oder n-Heptylrest. Verzweigte Alkylreste sind der Isopropyl, sek.-Butyl-(2-Methylpropyl-), 3-Methylbutyl-, 2,2-Dimethylpropyl-, 2-Methylpentyl- und 3,3-Dimethylbutylrest.

Soweit unter $R_1$ und $R_2$ Alkenyl verstanden wird, kommt ebenfalls sowohl verzweigtes als auch unverzweigtes, vorzugsweise unverzweigtes Alkenyl in Frage. Unverzweigte Alkenylreste sind der Allyl-(2-Propenyl), 2-Butenyl-, 3-Butenyl-, 2-Pentenyl-, 3-Pentenyl-, 4-Pentenylrest, Ein verzweigter Alkenylrest ist z.B. der 2-Methyl-2-propenylrest.

In einer weiteren Variante können $R_1$ und $R_2$ auch Cyclo-Alkylreste mit vorzugsweise 3 bis 6 C-Atomen enthalten. Dabei ist der Cyclo-Alkylrest entweder direkt oder über einen Alkylenrest mit 1-3 C-Atomen, vorzugsweise 1 C-Atom mit dem jeweiligen N-Atom verbunden. Beispiele für solche Reste sind die Cyclopropyl-, Cyclobutyl-, Cyclopentyl-, Cyclohexylgruppe, die, sofern sie über einen Alkylenrest gebunden sind, vorzugsweise über eine Methylengruppe mit dem jeweiligen N-Atom verbunden sind.

Wie bereits angeführt, können $R_1$ und $R_2$ gleiche oder verschiedene Bedeutung besitzen. Bevorzugt wird, daß $R_1$ und $R_2$ gleiche Bedeutung besitzen, insbesondere für den Fall, daß $R_3$ und $R_4$ verschieden sind.

Soweit unter $R_3$ und $R_4$ in einer bevorzugten Variante Alkyl, insbesondere unverzweigtes Alkyl verstanden wird, so gelten die für $R_1$ und $R_2$ gemachten Angaben entsprechend. Bevorzugt enthalten $R_3$ und $R_4$ jeweils 1 bis 7, insbesondere 1 bis 4 Kohlenstoffatome.

$R_3$ und $R_4$ können prinzipiell gleiche oder verschiedene Bedeutung besitzen. Bevorzugt wird, daß $R_3$ und $R_4$ gleiche Bedeutung besitzen, insbesondere für den Fall, daß $R_1$ und $R_2$ verschieden sind.

Ein Sonderfall für $R_3 = R_4$ ist, daß $R_3$ und $R_4$ gemeinsam eine Alkylenkette $-(CH_2)_n-$ bilden. Dabei nimmt n vorzugsweise Werte von 3 bis 6, insbesondere 3 bis 5 an.

Unter die Verbindungen gemäß Formel (I) fallen Verbindungen der Formel (Ib) oder Verbindungen der Formel (Ic).

Verbindungen vom Typ (Ic) werden als symmetrisch substituierte Verbindungen bezeichnet, da an beiden N-Atomen der gleiche Substituent steht.

Die Verbindungen der Formel (I) stellen wertvolle Zwischenprodukte zur Herstellung von pharmakologisch besonders wirksamen Diazabicyclo-(3,3,1)-nonanen dar.

Die erfindungsgemäßen neuen Zwischenprodukte dienen zur Herstellung von neuen pharmakologisch wirksamen Verbindungen der allgemeinen Formel (VI)

(s. Formel VI)

worin $R_1$ , $R_2$ , $R_3$ und $R_4$ obige Bedeutung besitzen und welche sich durch ausgeprägte herzwirksame Eigenschaften auszeichnen. Diese neuen pharmakologisch wirksamen Diazabicyclo-(3,3,1)-nonane, ihre pharmakologischen Eigenschaften und ihre Herstellung aus den erfindungsgemäßen Zwischenprodukten sind in der europäischen Patentanmeldung EP-A-0 103 833 ausführlich beschrieben und beansprucht. Die Verbindungen der Formel (VI) werden durch Reduktion entsprechend $N_1$ ,$N_2$ -disubstituierter Tetraoxoverbindungen der Formel (I) erhalten.

Die 2,4,6,8-Tetraoxo-3,7-diazabicyclo-(3,3,1)-nonane der Formel (V) lassen sich in Analogie zu J. Amer. Chem. Soc. 80, 3915 (1958) darstellen durch Umsetzung der Dinitrile (III)

(s. Formel III)

oder der Mononitrile (IV)

(s. Formel IV)

mit hochprozentigen Säure-Wasser-Gemischen, wobei z.B. als Säuren Schwefelsäure und Phosphorsäure zu nennen sind.

Die Dinitrile der Formel (III) sind literaturbekannt oder können in Analogie zu bekannten Verfahren synthetisiert werden, indem man Alkylidenessigester mit Cyanacetamiden kondensiert (Org. Syn. 39, 52) oder Cyanessigester in ammoniakalischer Alkohol-Lösung mit Ketonen zur Reaktion bringt (Org. Syn 36, 28).

Die Mononitrile der Formel (IV), welche zum Teil literaturbekannt sind, werden erhalten durch Kondensation des entsprechenden Ketons mit Cyanacetamid im alkalischem Medium (J. Chem. Soc 99, 422 (1911)).

Die Alkylierung der Tetraoxoverbindungen (V) erfolgt unter basischen Bedingungen, beispielsweise mit Natriumhydrid in Dimethylformamid, Alkalicarbonat in Dimethylformamid, Natriummethylat in Methanol, Natriumisopropylat in Isopropanol, Natriumamid in Toluol oder Xylol, nach dem bekannten Prinzip der Phasentransferkatalyse, u.a. Vorzugsweise erfolgt die Alkylierung, mit Natriumhydrid oder Alkalicarbonat in Dimethylformamid bei erhöhter Temperatur.

Die Ausgangsmaterialien für die beschriebenen Verfahren werden vorzugsweise in stöchiometrischen Mengen eingesetzt. Bei der Alkylierung der Verbindungen (Ia) verwendet man vorzugsweise einen überschuß an Deprotonierungs- bzw. Alkylierungsmittel von 25 bis 70 %.

Als Alkylierungsmittel werden Verbindungen $R_6$ X verwendet, in denen X für eine an sich bekannte Fluchtgruppe steht. Insbesondere werden als Alkylierungsmittel die entsprechenden Alkylhalogenide, -tosylate, -brosylate oder -mesylate, vorzugsweise Alkylhalogenide, insbesondere -chloride oder -bromide eingesetzt. Es ist auch möglich, das Alkylierungsmittel in situ aus den entsprechenden Alkoholen zu erzeugen, z B. nach der Methode von Mitsunobu (Synthesis 1981, S.1), bei der ein separater Zusatz von Deprotonierungsmittel entfällt.

Die Umsetzungen können bei Normaldruck oder auch bei erhöhtem Druck durchgeführt werden, vorzugsweise bei Normaldruck.

Die Umsetzungen erfolgen vorzugsweise in inerten organischen Lösungsmitteln oder Gemischen derselben mit Wasser. Geeignete inerte organische Lösungsmittel sind beispielsweise Äther wie Diäthyläther, Dioxan oder Tetrahydrofuran, Halogenkohlenwasserstoffe wie Methylendichlorid oder Tetrachlorkohlenstoff, Dimethylformamid, Aceton.

Die Reaktionstemperaturen können bei den Verfahren in einem Bereich zwischen 20 und 200 C° varriieren, vorzugsweise zwischen 40 und 150 °C.

Für den Fall, daß $R_5$ in den Verbindungen der Formel (V) Wasserstoff darstellt, können die Verbindungen zunächst monoalkyliert werden oder dialkyiiert werden zu Verbindungen der Formel (Ic).

Nach dem angegebenen Verfahren gelingt es, die unterschiedlichsten Substitutionsmuster zu realisieren. So kann man insbesondere durch Wahl von $R_3$ und $R_4$ in den Ausgangsverbindungen (III) bzw. (IV) die symmetrische oder asymmetrische Substitution der 9-Stellung der resultierenden Diazabicyclo-(3,3,1)-nonane erreichen. Andererseits ist es auch leicht möglich, je nach Wahl der Alkylierungsmittel N,N'-symmetrisch oder -asymmetrisch substituierte Diazabicyclo-(3,3,1)-nonane zu erhalten.

Für den Fall, daß $R_1$ und $R_2$ sowie $R_3$ und $R_4$ jeweils verschieden sind, erhält man auf den verschiedenen Stufen ein Gemisch von Stereoisomeren. Die Trennung dieser Stereoisomeren gelingt nach an sich bekannten Methoden wie z.B. fraktionierte Salzfällung, Fraktionierung mittels Säulenchromatophraphie oder in wäßriger Lösung bei unterschiedlichem pH-Wert.

Die Reduktion der N,N'-dialkylierten 2,4,6,8-Tetraoxo-3,7-diazabicyclo-(3,3,1)-nonane der Formel (I) zu pharmakologisch aktiven N,N'-disubstituierten 3,7-Diazabicyclo-(3,2,1)-nonan-Verbindungen kann nach an sich bekannten Methoden (z.B. J Amer. Chem. Soc 78, 2582 (1956) oder Israel. J. Chem. 4 39 (1966)) erfolgen. Als besonders vorteilhaft erweist es sich, als Reduktionsmittel komplexe Metallhydride wie z.B. Lithiumaluminiumhydrid oder Natriumborhydrid in Gegenwart von Lewis-Säuren einzusetzen.

Vorzugsweise verwendet man Lithiumaluminiumhydrid in einem 70 zu 30 Gemisch von Tetrahydrofuran und Toluol. Im Gegensatz zu den oben angeführten Methoden oder zu Eur. Med. Chem 12, 301, (1977) erreicht man in diesem Lösungsmittelgemisch bereits nach relativ kurzen Reaktionszeiten von in der Regel 2 bis 4 Stunden eine vollständige Umsetzung. Ist der Zutritt des Reduktionsmittels aus sterischen Gründen behindert, wie z. B. bei N-i-Propyl-Substitution, so sind längere Reaktionszeiten erforderlich. Es ist vorteilhaft einen Überschuß von 200 bis 400 % an Reduktionsmittel einzusetzen.

Sofern Reste $R_1$ ungesättigten Charakter besitzen, also Alken bedeuten, wird als Reduktionsmittel vorzugsweise Natrium-bis-(2-methoxyethoxy)-dihydroaluminat verwendet.

Die so erhaltenen Verbindungen der Formel (VI) und ihre pharmazeutisch verwendbaren Säureadditionssalze zeichnen sich durch interessante herzwirksame, pharmakolgische Eigenschaften aus und zeigen insbesondere sauerstoffeinsparende, die Herzfrequenz beeinflussende und herzrhythmisierende Wirkungen. Dabei zeigen sie schon in geringen Dosen eine befriedigende antiarrhythmische Wirkung. Darüber hinaus ist eine unerwünschte negative Beeinflussung des Kontraktionsvermögens des Herzens äußerst gering.

Die nachfolgenden Beispiele sollen die Herstellung der neuen Verbindungen naher erläutern.

Beispiel 1

Allgemeine Vorschrift zur Cyclisierung zu den Tetraoxo-3,7-Diazabicyclo-(3,3,1)-nonanen der Formel (V):

20 g Dinitril der allgemeinen Formel (III) (hergestellt analog zu Org. Syn. 39, 52) bzw. 20 g Mononitril der allgemeinen Formel (IV) (hergestellt analog zu J. Chem. Soc. 99, 422 (1911)) werden in etwa 100 ml Säure unter Rühren bei 120 °C bis 140 °C bis zur vollständigen Auflösung erhitzt. Nach etwa 10 bis 15 Minuten gießt man den Kolbeninhalt auf Eiswasser. Die ausfallende Tetraoxoverbindung (V) wird abgesaugt, gegebenenfalls umkristallisiert (vorzugsweise aus Äthanol) und getrocknet. Nach dieser Arbeitsweise wurden die in Tabelle 1a und 1b angegebenen Verbindungen erhalten.

Tabelle 1 a: Herstellung von Tetraoxoverbindungen der Formel (V) mit $R_5 = H$

| Nr. | $R^3$ | $R^4$ | Säure [Vol %] | Schmelzpunkt [°C] |
|---|---|---|---|---|
| 101 x) | $CH_3$ | $CH_3$ | 60 % $H_2SO_4$ | über 350 |
| 102 | $C_2H_5$ | $C_2H_5$ | 60 % $H_2SO_4$ | 230 |
| 103 | $n-C_3H_7$ | $n-C_3H_7$ | 60 % $H_2SO_4$ | 190 |
| 104 | $n-C_4H_9$ | $n-C_4H_9$ | 85 % $H_2SO_4$ | 195 - 199 |
| 105 | $-(CH_2)_3-$ | | 60 % $H_2SO_4$ | über 350 |
| 106 x) | $-(CH_2)_4$ | | 60 % $H_2SO_4$ | über 350 |
| 107 x) | $-(CH_2)_5-$ | | 60 % $H_2SO_4$ | 310 |
| 108 x) | $CH_3$ | $C_2H_5$ | $H_2SO_4/H_3PO_4$ 1:1 | 324 - 26 |
| 109 x) | $CH_3$ | $n-C_3H_7$ | $H_2SO_4/H_3PO_4$ 1:1 | 275 |
| 110 | $C_2H_5$ | $n-C_4H_9$ | 70 % $H_2SO_4$ | 140 |

x) nicht erfindungsgemäß

Tabelle 1 b: Herstellung von Tetraoxoverbindungen der Formel (V) mit $R_5 \neq H$

| Nr. | $R^5$ | $R^3$ | $R^4$ | Säure [Vol %] | Schmelz-pkt. [°C] |
|---|---|---|---|---|---|
| 150 | $n-C_4H_9$ | $CH_3$ | $CH_3$ | $H_2SO_4/H_3PO_4$ 1:1 | 175-178 |
| 151 | $-CH_2-CH(CH_3)_2$ | $n-C_3H_7$ | $n-C_3H_7$ | $H_2SO_4/H_3PO_4$ 1:1 | 149-153 |
| 152 | $-CH_2-$ 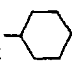 | $n-C_4H_9$ | $n-C_4H_9$ | 60 % $H_2SO_4$ | 140 |
| 153 | $n-C_6H_{13}$ | $C_2H_5$ | $C_2H_5$ | $H_2SO_4/H_3PO_4$ 1:1 | Öl |
| 154 | $-CH_2-CH(CH_3)_2$ | $-(CH_2)_5-$ | | $H_2SO_4/H_3PO_4$ 1:1 | 182 |

## Beispiel 2

Allgemeine Vorschrift zur Alkylierung der Tetraoxoverbindungen (V)

Beispiel 2a:

Dialkylierung der Tetraoxoverbindungen (V) ($R_5$ = H) mittels $R_6$ X zu Verbindungen der Formel (Ic)

0,1 mol der Tetraoxoverbindung (V), die gemäß Beispiel 1 hergestellt wurde, wird in einem ausgeheizten Dreihalskolben eingewogen, mit 200 ml absolutem Dimethylformamid versetzt und auf 60 bis 70 °C erwärmt. Portionsweise gibt man 0,25 mol NaH (berechnet als 100%ig) hinzu und kocht den Ansatz 1 Stunde lang unter Rückfluß. Nach dem Abkühlen der Reaktionslösung tropft man 0,3 mol des in 50 ml absoluten Dimethylformamid gelösten Alkylierungsmittels schnell hinzu und hält den Ansatz dann weitere 3 Stunden am Rückfluß. Der Hauptteil des Lösungsmittels wird im Vakuum abgezogen. Den Rückstand versetzt man mit Methylenchlorid und wäscht mit 20%iger Natriumhydroxidlösung. Die wäßrige Phase wird nochmals mit Methylenchlorid extrahiert. Nach Vereinigen der organischen Phasen werden diese mehrmals mit Wasser gewaschen und über Magnesiumsulfat getrocknet. Der nach Abdestillieren des Lösungsmittels verbleibende Rückstand wird aus Äther/Hexan umkristallisiert.

Alternativ wurde die Alkylierung anstatt mit NaH mit Alkalicarbonat als Base wie folgt durchgeführt:

0,1 mol der Tetraoxoverbindung (V), die gemäß Beispiel 1 hergestellt wurde, wird in einem ausgeheizten Dreihalskolben eingewogen, mit 0,25 mol Alkalicarbonat und 200 ml absolutem Dimethylformamid versetzt und für 1 Stunde auf 120 ° C erhitzt. Nach dem Abkühlen der Reaktionslösung tropft man 0,3 mol des in 50 ml abs. Dimethylformamid gelösten Alkylierungsmittels schnell hinzu und hält den Ansatz bis zur vollständigen Umsetzung am Rückfluß. Nach der üblichen Aufarbeitung wird der verbleiberde Rückstand aus Äther/Hexan umkristallisiert.

In der Tabelle 2a werden einige der nach diesem Beispiel hergestellten Verbindungen charakterisiert. In der Spalte "Base" bedeuten dabei "A" NaH und "C" Alkalicarbonat.

Beispiel 2b:

Allgemeine Vorschrift zur Monoalkylierung der Tetraoxoverbindungen (V) ($R_5$ = H) mittels $R_6$ X zu Verbindungen der Formel (Ib).

Es wird wie in Beispiel 2a verfahren, wobei nur die jeweils halbe Menge an NaH bzw. Alkalicarbonat sowie Alkylierungsmittel zum Einsatz kommt.

Tabelle 2a: Dialkylierung der Tetraoxoverbindungen (V) ($R_5$ = H) mittels $R_6X$ zu Verbindungen der Formel (Ic)

| Nr. | $R^1$ | $R^2$ | $R^3$ | $R^4$ | X | Base | Schmelzpunkt [°C] |
|---|---|---|---|---|---|---|---|
| 201 | $C_2H_5$ | $C_2H_5$ | $CH_3$ | $CH_3$ | Br | A | 152-153 |
| 202 | $n-C_4H_9$ | $n-C_4H_9$ | $CH_3$ | $CH_3$ | Br | C | 97 |
| 203 | $n-C_6H_{13}$ | $n-C_6H_{13}$ | $CH_3$ | $CH_3$ | Br | A | 68-69 |
| 204 | $CH_2=CH$<br>$-CH_2-CH_2$ | $CH_2=CH$<br>$-CH_2-CH_2$ | $CH_3$ | $CH_3$ | Br | C | 91 |
| 205 | $i-C_3H_7$ | $i-C_3H_7$ | $CH_3$ | $CH_3$ | Br | A | 99-101 |
| 206 | $n-C_{10}H_{21}$ | $n-C_{10}H_{21}$ | $CH_3$ | $CH_3$ | Br | A | 63 |
| 207 | $n-C_3H_7$ | $n-C_3H_7$ | $C_2H_5$ | $C_2H_5$ | Cl | A | 94 |
| 208 | $i-C_3H_7$ | $i-C_3H_7$ | $n-C_3H_7$ | $n-C_3H_7$ | Br | A | 145-147 |
| 209 | $CH_3$ | $CH_3$ | $n-C_4H_9$ | $n-C_4H_9$ | J | A | 135-137 |
| 210 | $n-C_4H_9$ | $n-C_4H_9$ | $n-C_4H_9$ | $n-C_4H_9$ | Br | A | 73- 75 |
| 211 | $n-C_6H_{13}$ | $n-C_6H_{13}$ | $-(CH_2)_4-$ | | Br | A | 70 |
| 212 | $-CH_2-$ 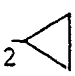 | $-CH_2-$ 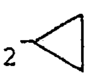 | $-(CH_2)_4-$ | | Cl | A | 160-161 |
| 213 | $-CH_2-$ 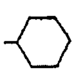 | $-CH_2-$ 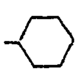 | $-(CH_2)_5-$ | | Br | A | 140 |
| 214 | $CH_2=CH$<br>$-CH_2-CH_2$ | $CH_2=CH$<br>$-CH_2-CH_2$ | $-(CH_2)_5-$ | | Br | C | 120 |

Tabelle 2a : Fortsetzung

| Nr. | $R^1$ | $R^2$ | $R^3$ | $R^4$ | X | Base | Schmelzpunkt [°C] |
|---|---|---|---|---|---|---|---|
| 215 | $n-C_4H_9$ | $n-C_4H_9$ | $CH_3$ | $C_2H_5$ | Br | A | 82 |
| 216 | $-CH_2-$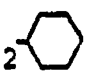 | $-CH_2-$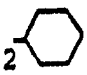 | $CH_3$ | $C_2H_5$ | Br | A | 112-115 |
| 217 | $i-C_3H_7$ | $i-C_3H_7$ | $CH_3$ | $n-C_3H_7$ | Br | A | 103 |
| 218 | $CH_2{=}CH$<br>$-CH_2-CH_2$ | $CH_2{=}CH$<br>$-CH_2-CH_2$ | $CH_3$ | $n-C_3H_7$ | Br | A | 57 |
| 219 | $n-C_6H_{13}$ | $n-C_6H_{13}$ | $C_2H_5$ | $n-C_4H_9$ | Br | A | Öl |
| 220 | $CH_2{=}CH$<br>$-CH_2-CH_2$ | $CH_2{=}CH$<br>$-CH_2-CH_2$ | $CH_3$ | $CH_3$ | Br | A | 128-130 |
| 221 | $n-C_4H_9$ | $n-C_4H_9$ | $-(CH_2)_3-$ | | Br | A | 110 |

Tabelle 2b: Monoalkylierung der Tetraoxoverbindungen (V) ($R_5 \neq H$) mittels $R_6X$ zu Verbindungen der Formel (Ib)

| Nr. | $R^1$ | $R^2$ | $R^3 = R^4$ | X | Base | Schmelz-pkt. [°C] |
|---|---|---|---|---|---|---|
| 250 | $n-C_4H_9$ | $-CH_2-CH(CH_3)_2$ | $CH_3$ | Br | A | 80 |
| 251 | $n-C_4H_9$ | 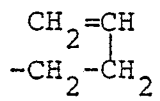 | $CH_3$ | Br | A | 80-83 |
| 252 | $n-C_4H_9$ | $-CH_2-$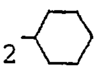 | $CH_3$ | Br | A | 100 |
| 253 | $CH_3$ | $n-C_6H_{13}$ | $C_2H_5$ | J | A | 93-96 |
| 254 | $i-C_3H_7$ | $-CH_2-$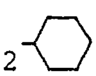 | $n-C_4H_9$ | Br | A | 85 |
| 255 | $-CH_2-CH(CH_3)_2$ | 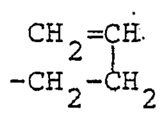 | $n-C_3H_7$ | Br | A | Öl |
| 256 | $i-C_3H_7$ | $-CH_2-CH(CH_3)_2$ | $-(CH_2)_4-$ | Br | A | 101 |

Beispiel 3

Allgemeine Vorschrift zur Reduktion der dialkylierten Tetraoxoverbindungen zu den 3,7-Diazabicyclo-(3,3,1)-nonanen der Formel (VI).

In einem ausgeheizten Dreihalskolben befinden sich 0,1 mol Lithiumaluminiumhydrid in 100 ml einer Lösung aus 70 ml absolutem Tetrahydrofuran und 30 ml absolutem Toluol. Bei 80 °C ölbadtemperatur tropft man langsam 0,025 mol Tetraoxoverbindung in 100 ml einer 70:30-Mischung von Tetrahydrofuran : Toluol zu.

Man hält den Ansatz 2 bis 4 Stunden lang auf 120 °C und hydrolysiert anschließend unter basischen Bedingungen. Nach Extraktion mit Methylenchlorid und Trocknen der organischen Phase mit Magnesiumsulfat engt man die Lösung ein. Mit Hilfe der Kugelrohrdestillation unter vermindertem Druck werden die 3,7-Diazabicyclo-(3,3,1)-nonane erhalten.

N,N'-Disubstituierte Tetraoxoverbindungen mit N-Alkenylsubstituenten werden analog mit Natrium-bis(2-methoxyethoxy)-dihydroaluminat (Handelsname Red-Al[R]) in Toluol als Reduktionsystem reduziert.

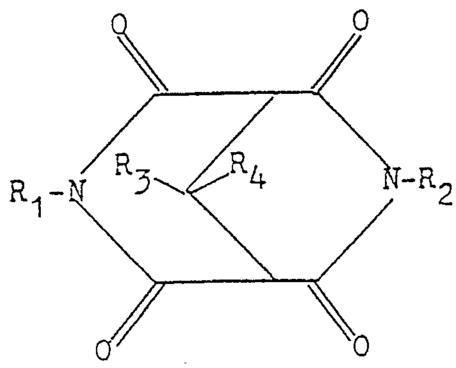

(I)

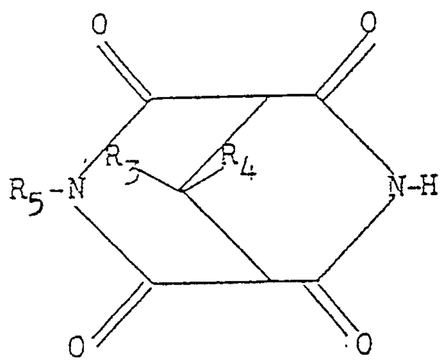

( V)

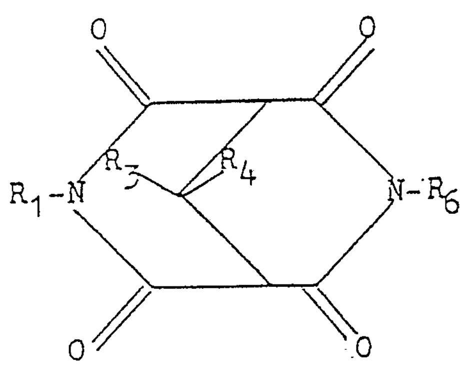

(Ib)

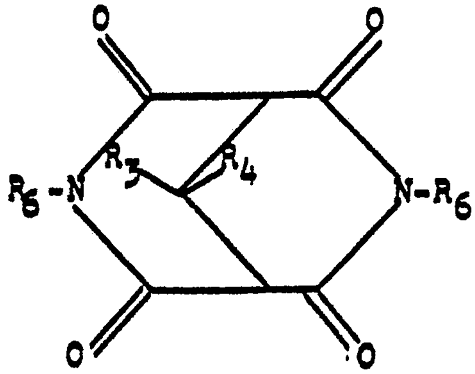

(Ic)

$R_6$-X (II

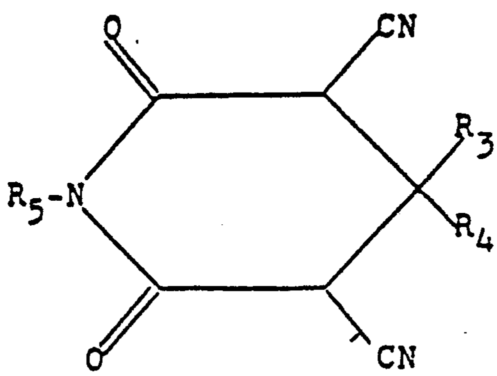

(III)

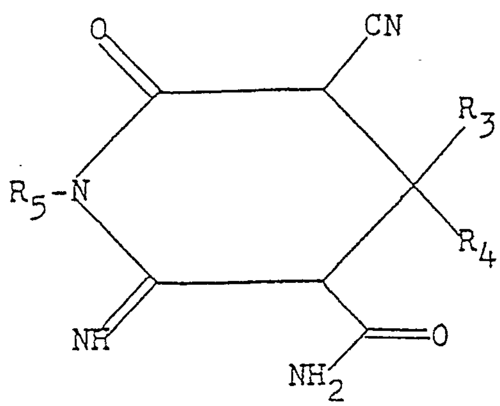

(IV)

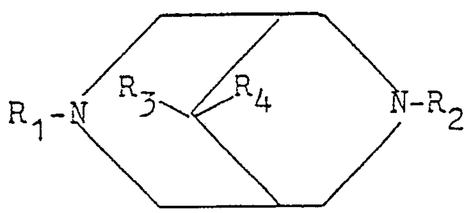

(VI)

**Patentansprüche**
**Patentansprüche für folgende Vertragsstaaten: BE, CH, DE, FR, GB, IT, LI, LU, NL, SE**

1. Tetraoxodiazabicyclononan-Verbindungen der allgemeinen Formel (I)

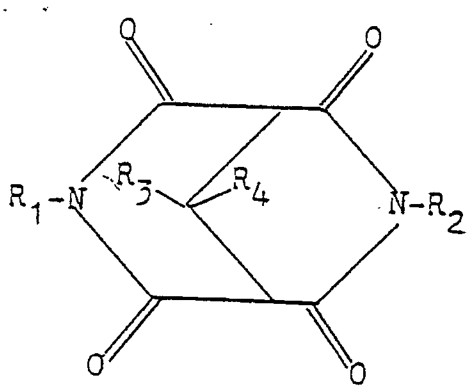

(I)

in der
$R_1$ und $R_2$ unabhängig voneinander Alkyl mit 1 bis 12 C-Atomen, Alkenyl mit 2 bis 12 C-Atomen oder Cycloalkyl mit 3 bis 6 C-Atomen, wobei das Cycloalkyl entweder direkt oder über einen Alkylenrest mit

1 bis 3 C-Atomen mit dem N-Atom verbunden ist, bedeuten und
$R_3$ und $R_4$ entweder unabhängig voneinander Alkyl mit 1 bis 7 C-Atomen bedeuten oder gemeinsam eine Alkylenkette $-(CH_2)_n-$ bilden, mit n = 3 bis 6, mit der Maßgabe, daß die Reste $R_1$ bis $R_4$ zusammengezählt mindestens 5 Kohlenstoffatome enthalten.

2. Verbindungen nach Anspruch 1, in denen $R_1$ und/oder $R_2$ für eine Alkyl-, Cycloalkyl- oder Cycloalkylalkylgruppe mit bis zu 7-C-Atomen stehen.

3. Verbindungen nach einem der Ansprüche 1 und 2, in denen die Reste $R_1$ und $R_2$ gleiche Bedeutung besitzen.

4. Verbindungen nach einem der vorhergehenden Ansprüche in denen $R_3$ und $R_4$ jeweils für Alkyl mit bis zu 4 Kohlenstoffatomen stehen.

5. Verbindungen nach einem der vorhergehenden Ansprüche, in denen $R_3$ und $R_4$ gleiche Bedeutung besitzen.

6. Verbindungen nach einem der Ansprüche 1 bis 3, in denen $R_3$ und $R_4$ gemeinsam eine Alkylenkette $-(CH_2)_n-$ bilden mit n = 3 bis 5.

**Patentansprüche für folgenden Vertragsstaat: AT**

1. Verfahren zur Herstellung von Tetraoxodiazabicyclononan-Verbindungen der allgemeinen Formel (I)

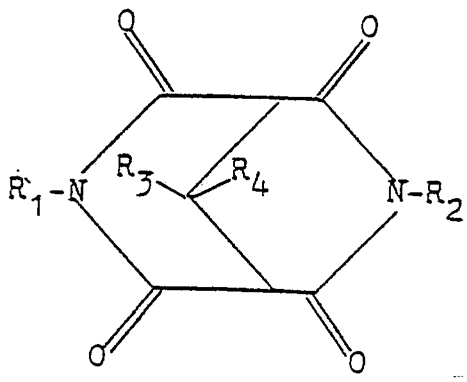

(I)

in der
$R_1$ und $R_2$ unabhängig voneinander Alkyl mit 1 bis 12 C-Atomen, Alkenyl mit 2 bis 12 C-Atomen oder Cycloalkyl mit 3 bis 6 C-Atomen, wobei das Cycloalkyl entweder direkt oder über einen Alkylenrest mit 1 bis 3 C-Atomen mit dem N-Atom verbunden ist, bedeuten und
$R_3$ und $R_4$ entweder unabhängig voneinander Alkyl mit 1 bis 7 C-Atomen bedeuten oder gemeinsam eine Alkylenkette $-(CH_2)_n-$ bilden, mit n = 3 bis 6, mit der Maßgabe, daß die Reste $R_1$ bis $R_4$ zusammengezählt mindestens 5 Kohlenstoffatome enthalten,
dadurch gekennzeichnet, daß man substituierte Dinitrile (III)

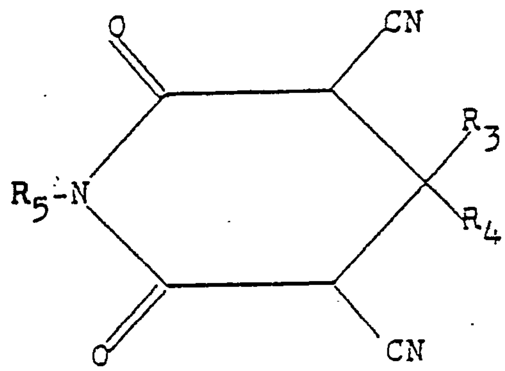

(III)

oder Mononitrile (IV)

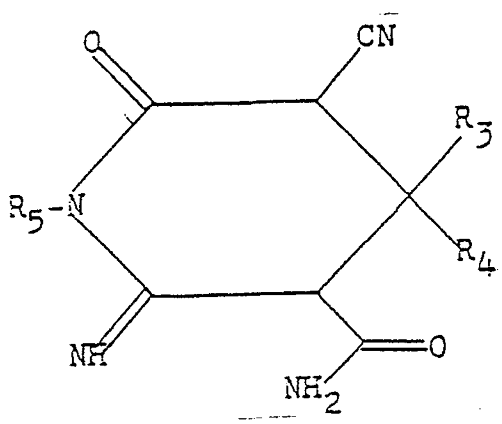

(IV)

in denen $R_3$ und $R_4$ obige Bedeutung besitzen und $R_5$ die für $R_1$ angegebene Bedeutung besitzt oder Wasserstoff bedeutet, sauer hydrolysiert zu den Verbindungen (V)

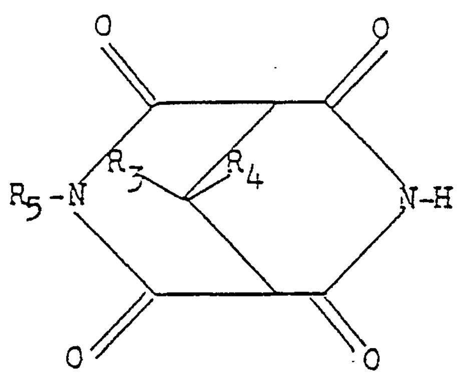

(V)

worin $R_5$ , $R_3$ und $R_4$ obige Bedeutung besitzen, und diese anschließend für den Fall, daß $R^5$ die Bedeutung von $R^1$ hat, mit Verbindungen der allgemeinen Formel (II)

$R_6$-X (II)

worin $R_6$ die für $R_1$ und $R_2$ angegebene Bedeutung hat und X eine Fluchtgruppe bedeutet, umsetzt zu Verbindungen der allgemeinen Formel (Ib)

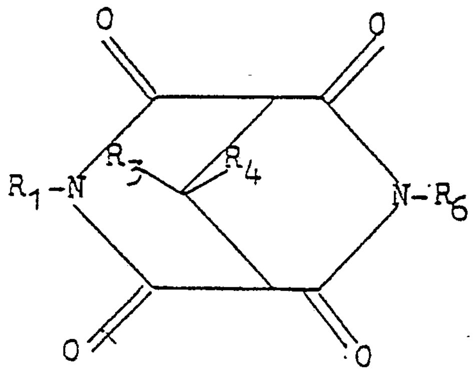

(Ib)

worin $R_1$ , $R_3$ , $R_4$ und $R_6$ obige Bedeutung besitzen,
oder
für den Fall, daß $R_5$ in den Verbindungen (V) Wasserstoff ist, diese Verbindungen mit Verbindungen der Formel (II) umsetzt zu symmetrisch substituierten Verbindungen (Ic)

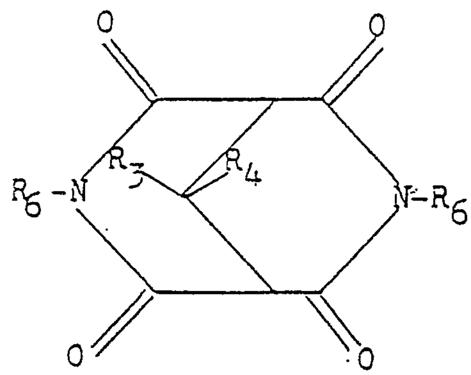

(Ic)

worin $R_3$ , $R_4$ und $R_6$ obige Bedeutung besitzen.

2. Verfahren nach Anspruch 1, dadurch gekennzeichnet, daß Verbindungen hergestellt werden, in denen $R_1$ und/oder $R_2$ für eine Alkyl-, Cycloalkyl- oder Cycloalkylalkylgruppe mit bis zu 7-C-Atomen stehen.

3. Verfahren nach einem der Ansprüche 1 und 2, dadurch gekennzeichnet, daß Verbindungen hergestellt werden, in denen die Reste $R_1$ und $R_2$ gleiche Bedeutung besitzen.

4. Verfahren nach einem der vorhergehenden Ansprüche, dadurch gekennzeichnet, daß Verbindungen hergestellt werden, in denen $R_3$ und $R_4$ jeweils für Alkyl mit bis zu 4 Kohlenstoffatomen stehen.

5. Verfahren nach einem der vorhergehenden Ansprüche, dadurch gekennzeichnet, daß Verbindungen hergestellt werden, in denen $R_3$ und $R_4$ gleiche Bedeutung besitzen.

6. Verfahren nach einem der Ansprüche 1 bis 3, dadurch gekennzeichnet, daß Verbindungen hergestellt werden, in denen $R_3$ und $R_4$ gemeinsam eine Alkylenkette $-(CH_2)_n$ -bilden mit n = 3 bis 5.

**Claims**

**Claims for the following Contracting States: BE, CH, DE, FR, GB, IT, LI, LU, NL, SE**

1. Tetraoxodiazabicyclononane compounds of the general Formula (I),

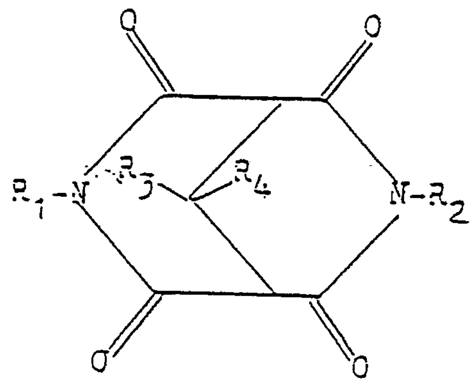

(I)

in which
$R_1$ and $R_2$, independently of each other, are alkyl with 1 to 12 C atoms, alkenyl with 2 to 12 C atoms or cycloalkyl with 3 to 6 C atoms, with the cycloalkyl being linked to the N atom either directly or via an alkylene radical with 1 to 3 C atoms, and
$R_3$ and $R_4$ are either, independently of each other, alkyl with 1 to 7 C atoms or together form an alkylene chain $-(CH_2)n-$, with n = 3 to 6, with the proviso that the radicals $R_1$ to $R_4$, added together, contain at least 5 carbon atoms.

2. Compounds according to Claim 1, in which $R_1$ and/or $R_2$ stand for an alkyl, cycloalkyl or cycloalkylalkyl group with up to 7 C atoms.

3. Compounds according to one of Claims 1 and 2, in which the radicals $R_1$ and $R_2$ have the same meaning.

4. Compounds according to one of the preceding Claims, in which $R_3$ and $R_4$ each stand for alkyl with up to 4 carbon atoms.

5. Compounds according to one of the preceding Claims, in which $R_3$ and $R_4$ have the same meaning.

6. Compounds according to one of Claims 1 to 3, in which $R_3$ and $R_4$ together form an alkylene chain $-(CH_2)n-$ with n = 3 to 5.

**Claims for the following contracting State: AT**

1. Method for preparing tetraoxodiazabicyclononane compounds of the general Formula (I),

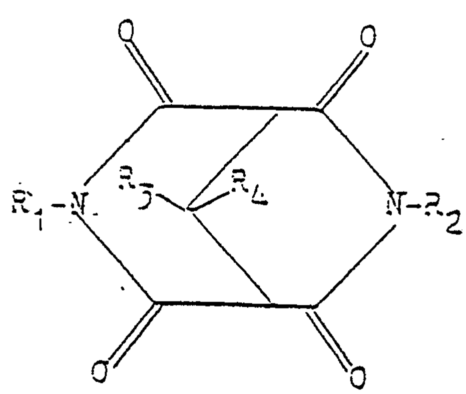

(I)

in which
$R_1$ and $R_2$, independently of each other, are alkyl with 1 to 12 C atoms, alkenyl with 2 to 12 C atoms or cycloalkyl with 3 to 6 C atoms, with the cycloalkyl being linked to the N atom either directly or via an alkylene radical with 1 to 3 C atoms, and

$R_3$ and $R_4$ are either, independently of each other, alkyl with 1 to 7 C atoms or together form an alkylene chain $-(CH_2)_n-$, with $n = 3$ to 6, with the proviso that the radicals $R_1$ to $R_4$, added together, contain at least 5 carbon atoms,
characterised in that substituted dinitriles (III),

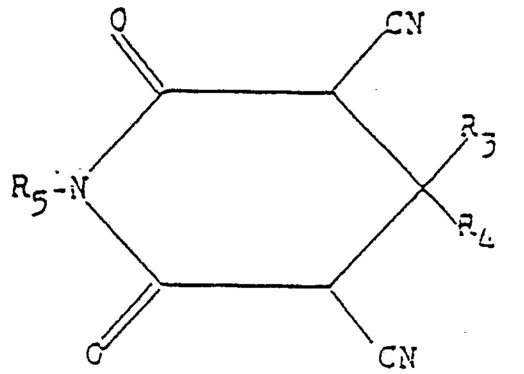

(III)

or mononitriles (IV),

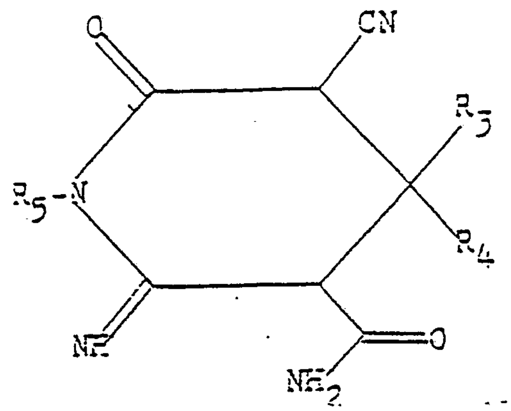

(IV)

in which $R_3$ and $R_4$ have the above meanings and $R_5$ has the meaning given for $R_1$ or is hydrogen, are subjected to acid hydrolysis to form the compounds (V),

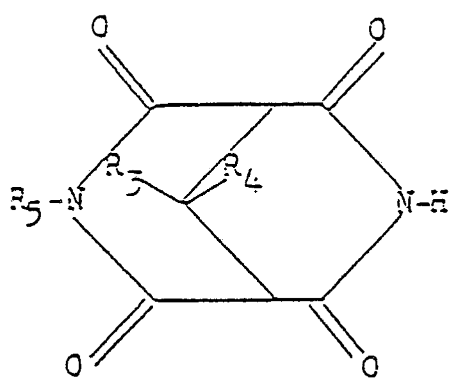

(V)

wherein $R_5$, $R_3$ and $R_4$ have the above meanings, and then, in the event that $R_5$ has the meaning of $R_1$, are reacted with compounds of the general Formula (II)

$R_6$-X (II)

wherein $R_6$ has the meaning given for $R_1$ and $R_2$ and X is a fugitive group, to form compounds of the general Formula (Ib),

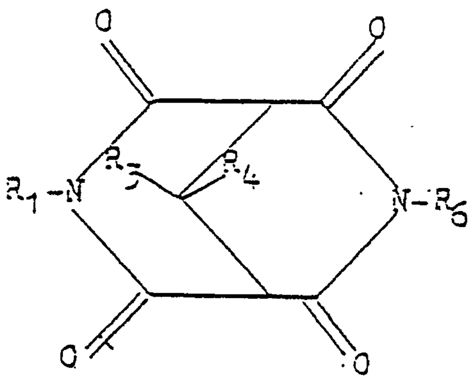

(Ib)

wherein $R_1$, $R_3$, $R_4$ and $R_6$ have the above meanings,
or,
in the event that $R_5$ in the compounds (V) is hydrogen, these compounds are reacted with compounds of the Formula (II) to form symmetrically substituted compounds (Ic),

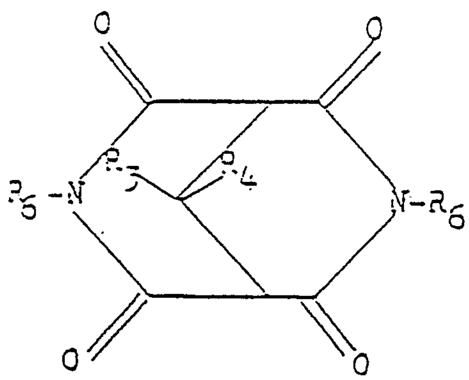

(Ic)

wherein $R_3$, $R_4$ and $R_6$ have the above meanings.

2. Process according to Claim 1, characterised in that compounds are prepared in which $R_1$ and/or $R_2$ stand for an alkyl, cycloalkyl or cycloalkylalkyl group with up to 7 C atoms.

3. Process according to one of Claims 1 and 2, characterised in that compounds are prepared in which the radicals $R_1$ and $R_2$ have the same meaning.

4. Process according to one of the preceding Claims, characterised in that compounds are prepared in which $R_3$ and $R_4$ each stand for alkyl with up to 4 carbon atoms.

5. Process according to one of the preceding Claims, characterised in that compounds are prepared in which $R_3$ and $R_4$ have the same meaning.

6. Process according to one of Claims 1 to 3, characterised in that compounds are prepared in which $R_3$ and $R_4$ together form an alkylene chain $-(CH_2)_n-$ with n = 3 to 5.

**Revendications**
**Revendications pour les Etats contractants suivants: BE, CH, DE, FR, GB, IT, LI, LU, NL, SE**

1. Composés de tétraoxodiazabicyclononane répondant à la formule générale (I)

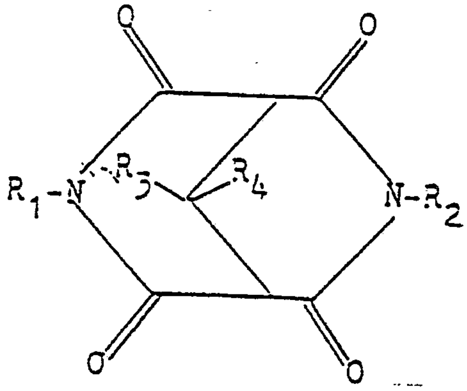

(I)

dans laquelle $R_1$ et $R_2$ signifient, indépendamment l'un de l'autre, un alkyle avec 1 à 12 atomes de C, un alcényle avec 2 à 12 atomes de C ou un cycloalkyle avec 3 à 6 atomes de C, le cycloalkyle étant lié à l'atome de N directement ou par l'intermédiaire d'un reste alkylène ayant 1 à 3 atomes de C, et $R_3$ et $R_4$ signifient, indépendamment l'un de l'autre, un alkyle avec 1 à 7 atomes de C ou forment ensemble une chaîne alkylène $-(CH_2)_n-$ avec $n = 3$ à 6, sous réserve que les restes $R_1$ à $R_4$ additionnés contiennent au moins 5 atomes de carbone.

2. Composés selon la revendication 1, dans lesquels $R_1$ et/ou $R_2$ représentent un groupe alkyle, cycloalkyle ou cycloalkylalkyle comportant jusqu'à 7 atomes de C.

3. Composés selon l'une des revendications 1 et 2, dans lesquels les restes $R_1$ et $R_2$ ont la même signification.

4. Composés selon l'une des revendications précédentes, dans lesquels $R_3$ et $R_4$ représentent chacun un alkyle comportant jusqu'à 4 atomes de carbone.

5. Composés selon l'une des revendications précédentes, dans lesquels $R_3$ et $R_4$ ont la même signification.

6. Composés selon l'une des revendications 1 à 3, dans lesquels $R_3$ et $R_4$ forment ensemble une chaîne alkylène $-(CH_2)_n-$ avec $n = 3$ à 5.

**Revendications pour l'Etat oontractant suivant: AT**

1. Procédé de préparation de composés de tétraoxodiazabicyclononane de formule générale (I)

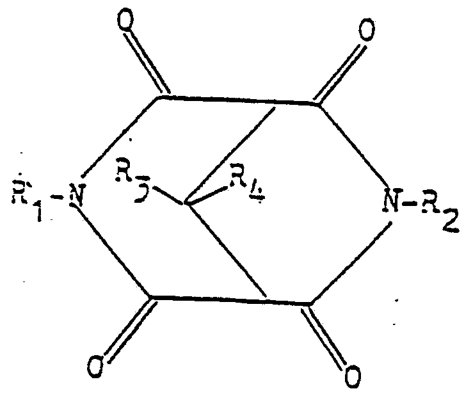

(I)

dans laquelle $R_1$ et $R_2$ signifient, indépendamment l'un de l'autre, un alkyle avec 1 à 12 atomes de C, un alcényle avec 2 à 12 atomes de C ou un cycloalkyle avec 3 à 6 atomes de C, le cycloalkyle étant lié à l'atome de N directement ou par l'intermédiaire d'un reste alkylène ayant 1 à 3 atomes de C, et $R_3$ et $R_4$ signifient, indépendamment l'un de l'autre, un alkyle avec 1 à 7 atomes de C ou forment

ensemble une chaîne alkylène $-(CH_2)_n-$ avec n = 3 à 6, sous réserve que les restes $R_1$ à $R_4$ additionnés contiennent au moins 5 atomes de carbone, caractérisé en ce que l'on soumet des dinitriles substitués (III)

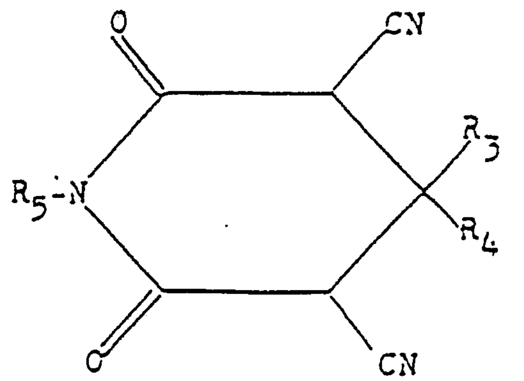

(III)

ou des mononitriles (IV)

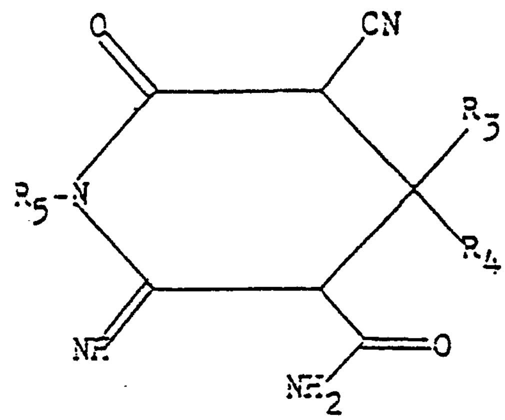

(IV)

dans lesquels $R_3$ et $R_4$ possèdent la signification précédente et $R_5$ a la signification indiquée à propos de $R_1$ ou signifie l'hydrogène, à une hydrolyse acide en vue de l'obtention des composés (V)

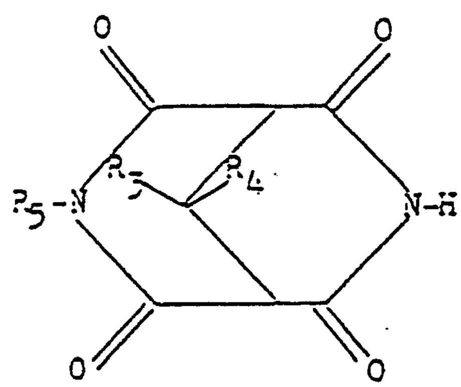

(V)

où $R_5$, $R_3$ et $R_4$ ont la signification précédente et que l'on fait ensuite réagir ceux-ci, au cas où $R_5$ a la signification de $R_1$, avec des composés de formule générale (II)

$R_6$-X (II)

dans laquelle $R_6$ a la signification indiquée à propos de $R_1$ et $R_2$ et X signifie un groupe partant , pour obtenir des composés de formule générale (Ib)

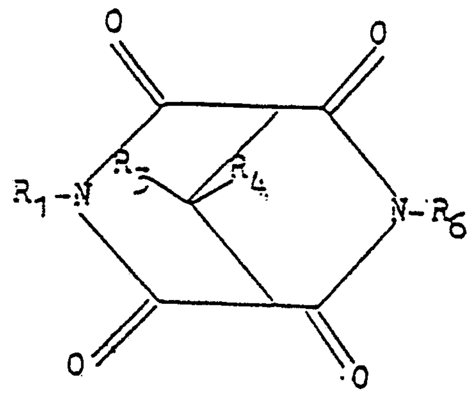

(Ib)

dans laquelle $R_1$, $R_3$, $R_4$ et $R_6$ ont la signification précédente ou que, au cas où $R_5$ dans les composés (V) est l'hydrogène, on fait réagir ces composés avec des composés de formule (II) pour obtenir des composés substitués symétriquement (Ic)

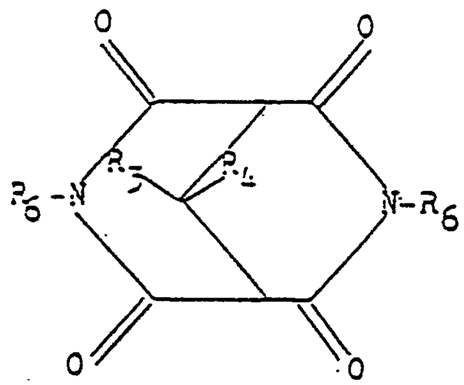

(Ic)

où $R_3$, $R_4$ et $R_6$ possèdent la signification précédente.

2. Procédé selon la revendication 1, caractérisé en ce qu'on prépare des composés dans lesquels $R_1$ et/ou $R_2$ représentent un groupe alkyle, cycloalkyle ou cycloalkylalkyle comportant jusqu'à 7 atomes de C.

3. Procédé selon l'une des revendications 1 et 2, caractérisé en ce qu'on prépare des composés dans lesquels les restes $R_1$ et $R_2$ ont la même signification.

4. Procédé selon l'une des revendications précédentes, caractérisé en ce qu'on prépare des composés dans lesquels $R_3$ et $R_4$ représentent chacun un alkyle comportant jusqu'à 4 atomes de carbone.

5. Procédé selon l'une des revendications précédentes, caractérisé en ce qu'on prépare des composés dans lesquels $R_3$ et $R_4$ ont la même signification.

6. Procédé selon l'une des revendications 1 à 3, caractérisé en ce qu'on prépare des composés dans lesquels $R_3$ et $R_4$ forment ensemble une chaîne alkylène $-(CH_2)_n-$ avec $n = 3$ à 5.